# EUROPEAN PATENT APPLICATION

(11) **EP 1 609 774 A1**
(43) Date of publication of application: **28.12.2005**
(21) Application number: 04014620.1
(22) Date of filing: 22.06.2004
(51) Int. Cl.: C07C 49/567, C07C 49/577, C07C 45/68

(54) **Fluorine atom containing benzyl ketone compound and process for producing the same**

(71) Applicant: DAINIPPON INK AND CHEMICALS, INC., Itabashi-ku Tokyo (JP)
(72) Inventor: Pithart, Cornelia, Dr., 14163 Berlin (DE); Doehler, Thomas, Dr., 16767 Leegebruch (DE); Berger, Daniel, Dr., 13507 Berlin (DE); Frings, Rainer Bruno, Dr., 12307 Berlin (DE); Lachowicz, Artur, Dr., 13467 Berlin (DE)
(74) Representative: Albrecht, Thomas

(57) **Abstract**

The invention relates to a fluorine atom containing benzyl ketone compound represented by the following formula (1), 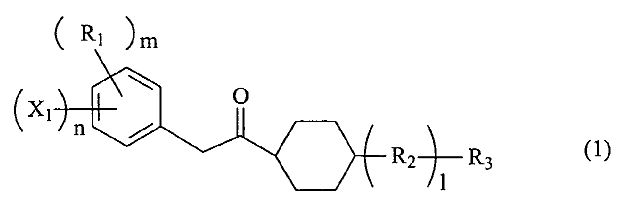 wherein R1 denotes methyl, ethyl, propyl, methoxy, or ethoxy, X1 denotes a fluorine atom or tri-fluoro methoxy, R2 denotes 1,4-cyclohexylene, or 1,4-phenylene, R3 denotes an aliphatic hydrocarbon group having 1 to 10 carbon atoms, 1 means an integer of 0 or 1, m means an integer of 0 or 1, and n means an integer of 1 to 3, which compound is quite useful as intermediate for the preparation of a liquid crystaline material. Furthermore, the compound can be obtained by a simple process of the present invention in high yield and selectivity, wherein the process comprises; reacting a fluorine atom containing benzyl haloid with an aliphatic hydrocarbon group containing cyclohexyl carbonyl chloride in the presence of Pd catalyst and zinc powder under an oxgen-free atmosphere.

## Description

### BACKGROUND OF THE INVENTION

### FIELD OF THE INVENTION

The present invention relates to a new fluorine atom containing benzyl ketone compound which is useful as an intermediate for a liquid crystal compound for an electro optical liquid crystal display material, and to a process for producing the ketone compound.

### DESCRIPTION OF RELATED ART

Liquid crystal display elements are now used not only in clocks and calculators, but also in various types of measuring instruments, automobile instrument panels, word processors, personal digital assistants, printers, computers and televisions. Liquid crystal compositions applied for the L.C. display elements comprise plural compounds, and many type of compounds have been synthesized so far.

Tetra-hydro naphthalene derivatives having Fluorine atom on its aromatic ring are well-known liquid crystalline compounds which are quite usable for active matrix liquid crystal display system.

Japanese laid-open patent No.2001-10991 discloses tetra-hydro naphthalene derivatives as liquid crystal materials which have good compatibility with the other compound.

However, although the tetra-hydro naphthalene derivatives have good properties such as optical and electrical anisotropy, serious difficulties had existed in that too many synthesis steps were necessary in order to produce the tetra-hydro naphthalene derivative, in particular, complicate and multiple steps were necessary for synthesis of a phenyl acetic acid which is conventionally used as a starting material.

Therefore, the complicate process had been an obstacle for the preparation of the tetra-hydro naphthalene derivatives so far, so that simple or concise methods to produce the tetra-hydro naphthalene derivatives have been desired in the liquid crystalline compound field.

From the viewpoint of simple or concise methods, the most preferable process for tetra-hydro naphthalene derivatives includes converting benzyl carbonyl compounds to tetra-hydro naphthalene compounds via Wittig Horner reaction.

However a fluorine atom substituted benzyl ketone compound as a starting material is difficult to synthesize. Because, conventional benzyl ketone compounds having no Fluorine atoms can be produced with Grignard reaction, on the contrary, in case of Fluorine atom substituted benzyl ketone compounds, the Fluorine atom causes inhibition of the formation of Grignard compound due to its electron attractive property. Further worse, if the Grignard compound should be obtained, the compound dimerizes quite easily, and the Grignard reaction does not take place any longer.

On the other hand, with respect to benzyl carbonyl synthesis, "CHEMISTRY LETTERS, pp. 1135-1138, 1981" discloses a simple method for the synthesis of (by) benzyl ketones by the reductive coupling of acyl chlorides and benzyl bromide by means of using zinc and a palladium catalyst.

However, the document only discloses using normal benzyl bromide having no fluorine atoms on the aromatic ring as a starting material, and has a clear defect of the lower yield of the objective compound in case of using branched aliphatic structure incorporated acyl chlorides, such as (CH3)2CHCOCl and (CH3)3CCOCl. Furthermore, by-products were inevitable due to the mild reaction conditions.

### SUMMARY OF THE INVENTION

It is therefore an object of the invention to provide a new fluorine atom containing benzyl ketone compound by a reaction of halogeno methyl fluorine atom substituted aromatic compounds with alicyclic acyl halide, which is quite useful as intermediate of a liquid crystalline material.

Another object is to provide a process for producing the new ketone compound, with high yield and high selectivity.

Unexpectedly, we have now found that selecting a combination of a Pd catalyst and zinc powder as a catalyst and controlling the reaction condition using an atmosphere wherein oxygen is excluded easily give high yield and high selectivity in spite of the presence of fluorine atoms on the aromatic ring and alicyclic structures in the chemical structure thereof, and completed the present invention.

Accordingly, the present invention provides a new fluorine atom containing benzyl ketone compound represented by the following formula (1), 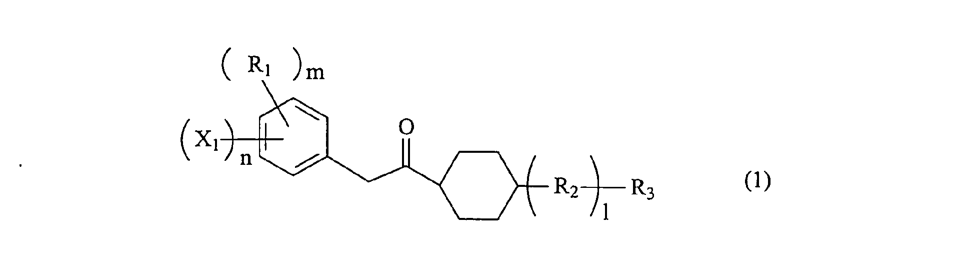 wherein R1 denotes methyl, ethyl, propyl, methoxy, or ethoxy, X1 a denotes fluorine atom or tri-fluoro methoxy, R2 denotes 1,4-cyclohexylene, or 1,4-phenylene, R3 denotes an aliphatic hydrocarbon group having 1 to 10 carbon atoms, 1 means an integer of 0 or 1, m means an integer of 0 or 1, and n means an integer of 1 to 3.

The present invention also provides a process for producing a fluorine atom containing benzyl ketone compound comprising: reacting a fluorine atom containing aryl methyl haloid with an C1 - C8 aliphatic hydrocarbon group containing cyclohexyl carbonyl chloride in the presence of Pd catalyst and zinc powder under an oxygen-free atmosphere.

According to the present invention, the fluorine atom containing benzyl ketone compound can be obtained by a simple method in high yield and selectivity and thereby the production of tetralin type liquid crystalline compounds in industrial scale is drastically improved.

### DETAILED DESCRIPTION OF THE PREFERRED EMBODIMENTS

The embodiments of the present invention will hereinafter be described in detail.

The new fluorine atom containing benzyl ketone compound of the present invention is represented by the following formula (1), 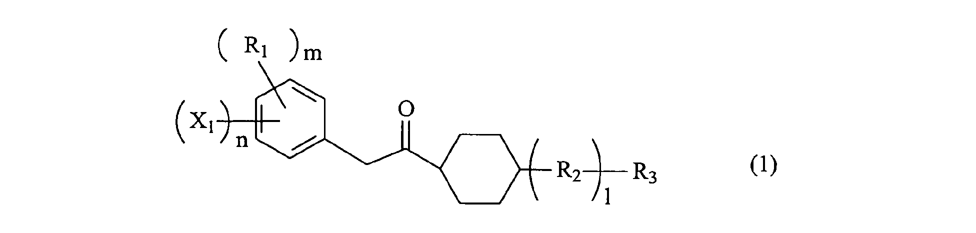 wherein R1 denotes methyl, ethyl, methoxy, or ethoxy, X1 denotes fluorine atom or tri-fluoro methoxy
R2 denotes 1,4-cyclohexylene, or 1,4-phenylene, R3 denotes an aliphatic hydrocarbon group having 1 to 20 carbon atoms, 1 means an integer of 0 or 1, m means an integer of 0 or 1, and n means an integer of 1 to 3.

Examples of the new fluorine atom containing benzyl ketone compound include several compounds such as benzyl ketone compounds having only fluorine atoms as substituents, benzyl ketone compounds having alkyl groups as well as fluorine atom as substituents,
benzyl ketone compounds having only tri-fluoro methoxy group as substituents, benzyl ketone compounds having alkoxy groups as well as fluorine atoms as substituents.

The examples of the benzyl ketone compound having only fluorine atoms as substituents are represented by the following structures, wherein R3 is defined in the formula (1).

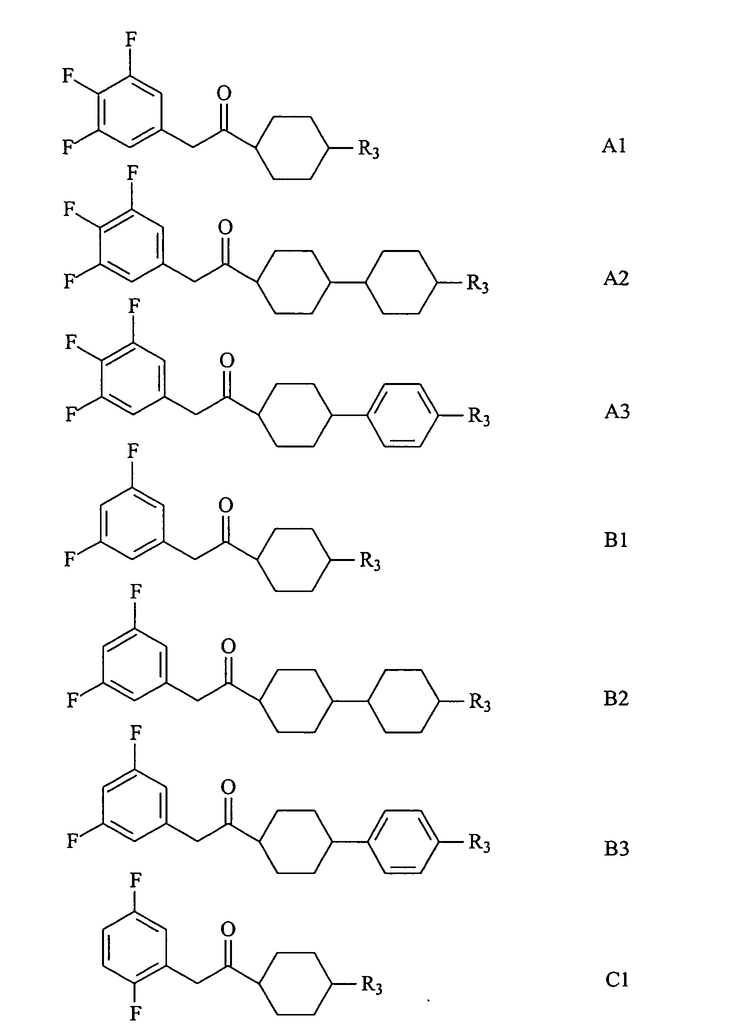

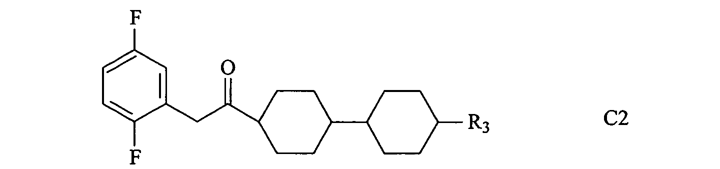

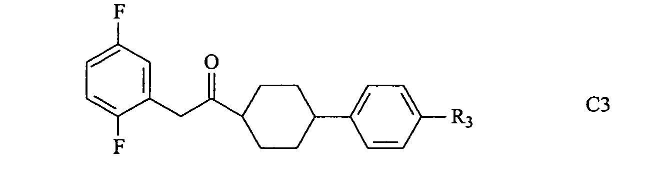

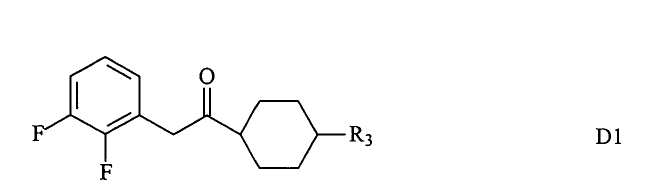

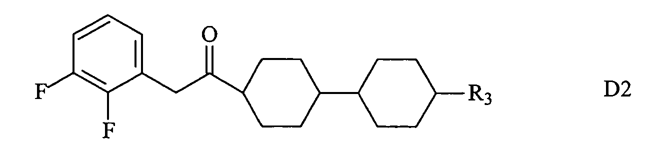

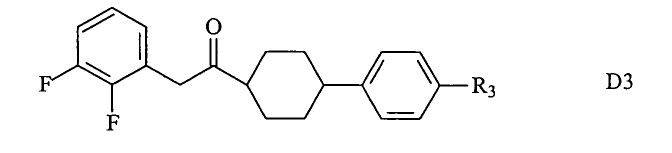

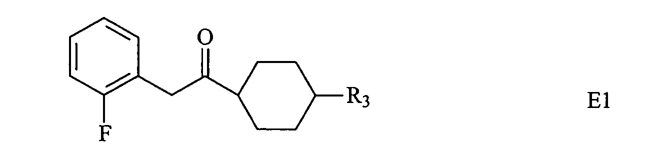

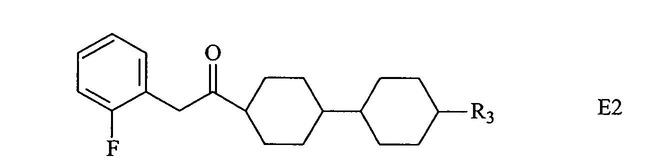

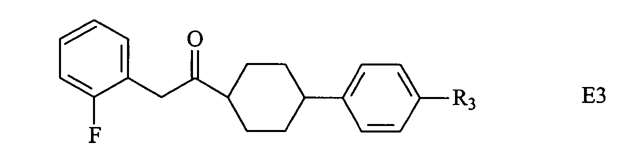

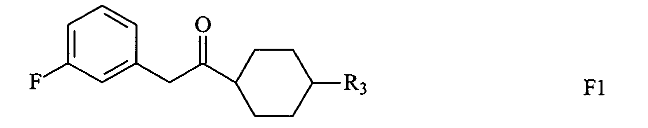

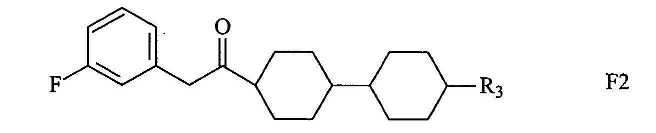

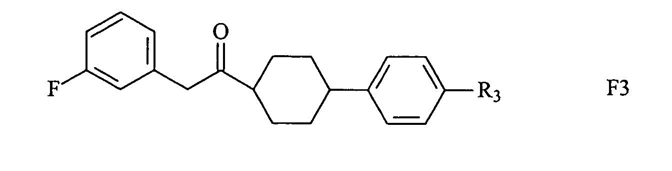

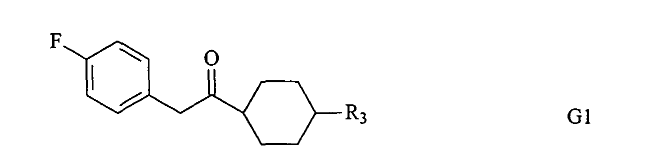

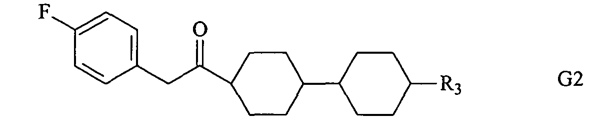

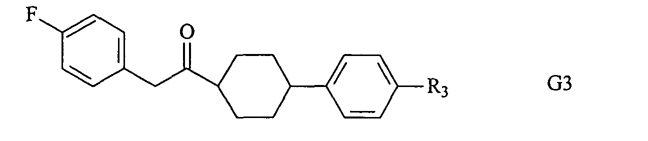

The examples of the benzyl ketone compound having alkyl groups as well as fluorine atoms as substituents are represented by the following structures, wherein R3 is defined as in the formula (1).

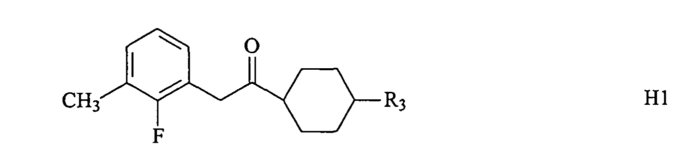

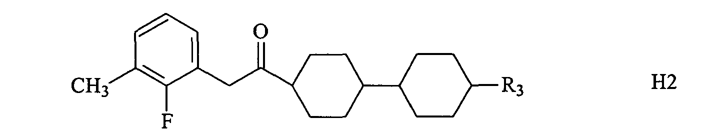

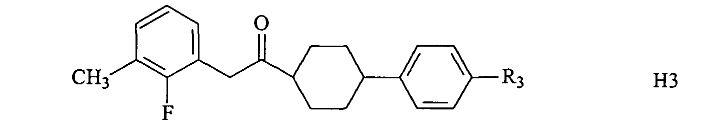

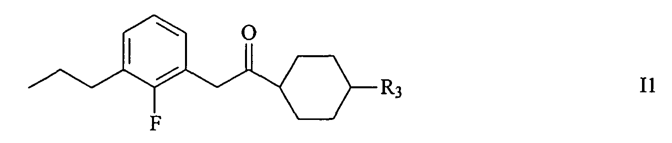

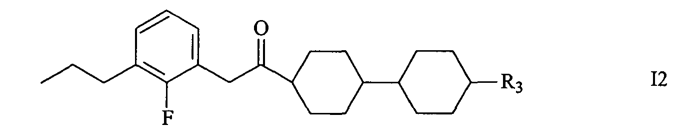

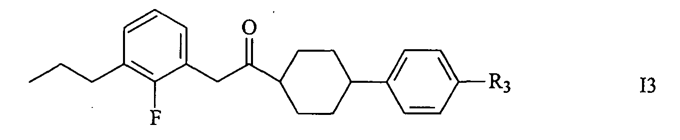

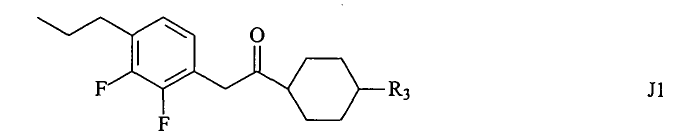

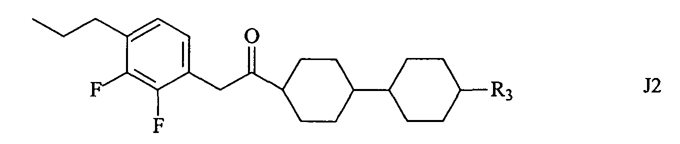

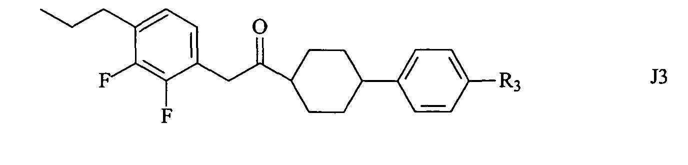

The examples of the benzyl ketone compound having only a tri-fluoro methoxy group as substituent are represented by the following structures, wherein R3 is defined as in the formula (1).

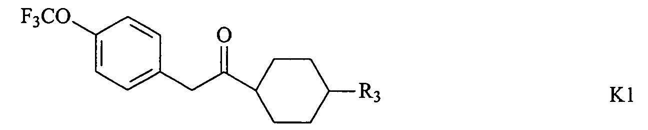

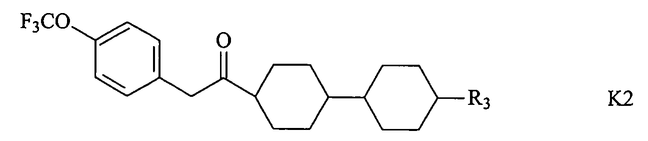

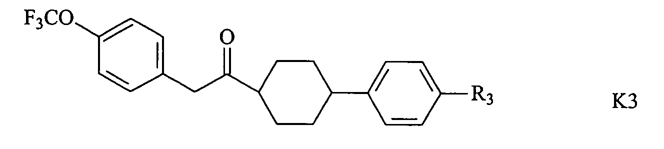

The examples of the benzyl ketone compound having alkoxy groups as well as fluorine atoms as substituents are represented by the following structures, wherein R3 is defined in the formula (1).

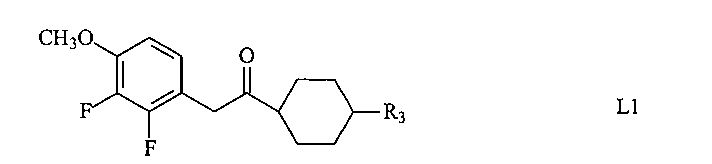

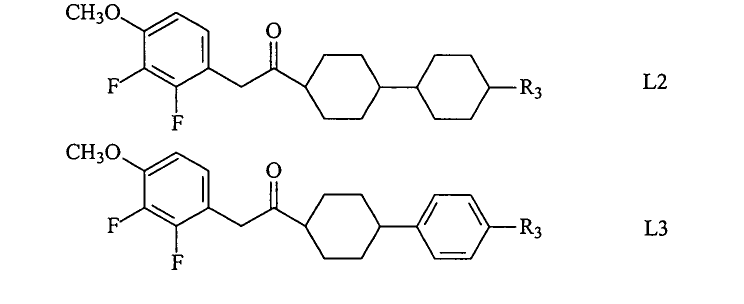

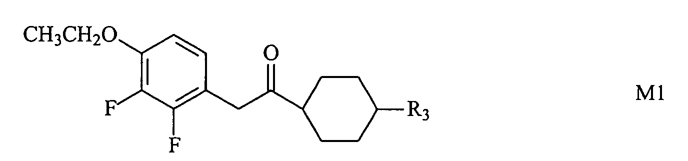

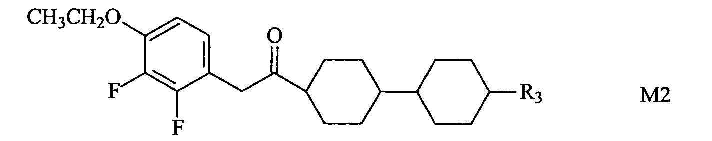

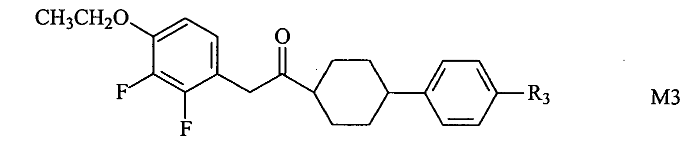

Here, R3 defined as an aliphatic hydrocarbon group having 1 to 10 carbon atoms in the aforementioned chemical structure (1) may be a linear or branched alkyl group, which includes methyl, ethyl, propyl, iso-propyl, n-butyl, t-butyl, pentyl, hexyl, cyclo-hexyl, n-heptyl, n-octyl. Among these structures, particularly methyl, or propyl is preferable from the viewpoint of anisotropy of the final compounds.

Among these structures, in case of using the compounds for a positive dielectric anisotropy (p-type) liquid crystalline compounds, the abovementioned A1 to A3, G1 to G3 and K1 to K3 are preferably used, in particular, the compounds represented by the following formula (2) are preferable due to the excellent anisotropy of the final liquid crystalline compound product thereof, 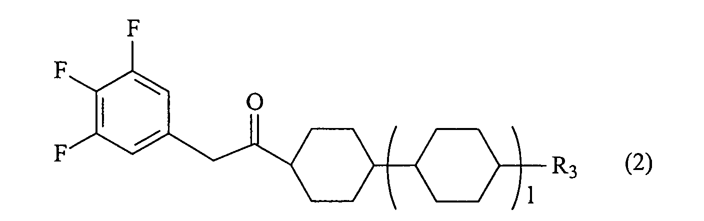 wherein R3 denotes an liniar or branched aliphatic hydrocarbon group having 1 to 10 carbon atoms, and l means an integer of 0 or 1.

Examples of the formula (2) include the group which consists of the aforementioned A1 to A3. Further, in particular, the compounds represented by the formula (2) are also preferable from the viewpoint of high yield and selectivity.

On the other hand, in case of using the compounds for negative dielectric anisotropy (n-type) liquid crystalline compounds, the abovementioned D1 to D3, F1 to F3, J1 to J3, L1 to L3, and M1 to M3 are preferably used, in particular, the compounds represented by the following formula (3) is preferable due to the excellent anisotropy of the liquid crystalline compound as a final product thereof, 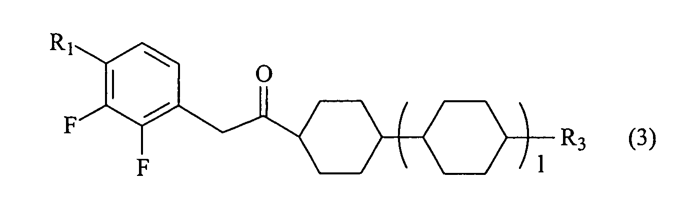 wherein R1 denotes hydrogen, methyl, ethyl, propyl, methoxy, or ethoxy,R3 denotes an aliphatic hydrocarbon group having 1 to 10 carbon atoms, and l means an integer of 0 or 1. Examples of the compound represented by the formula (3) include the group which consists of the aforementioned J1, J2, L1, L2, M1, and M2.

The above mentioned compounds can be prepared by the process of the present invention which comprises;
reacting a fluorine atom containing benzyl haloid with an cyclohexyl carbonyl chloride compound in the presence of Pd catalyst and zinc powder under an oxgen-freeatmosphere.

Suitable examples of the fluorine atom containing benzyl haloid, as a starting material of the present invention, are represented by the following general formula (4).

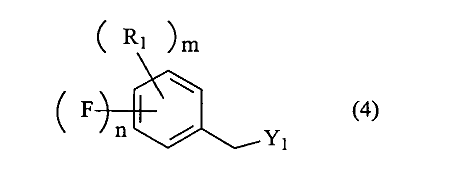

Here, R1, n, and m are defined as in formula (1), Y1 denotes halogen atoms such as chlorine and bromine.
Furthermore, specific examples of the compound of formula (4) include those represented by formula A-b-1 to M-b-2 shown below.

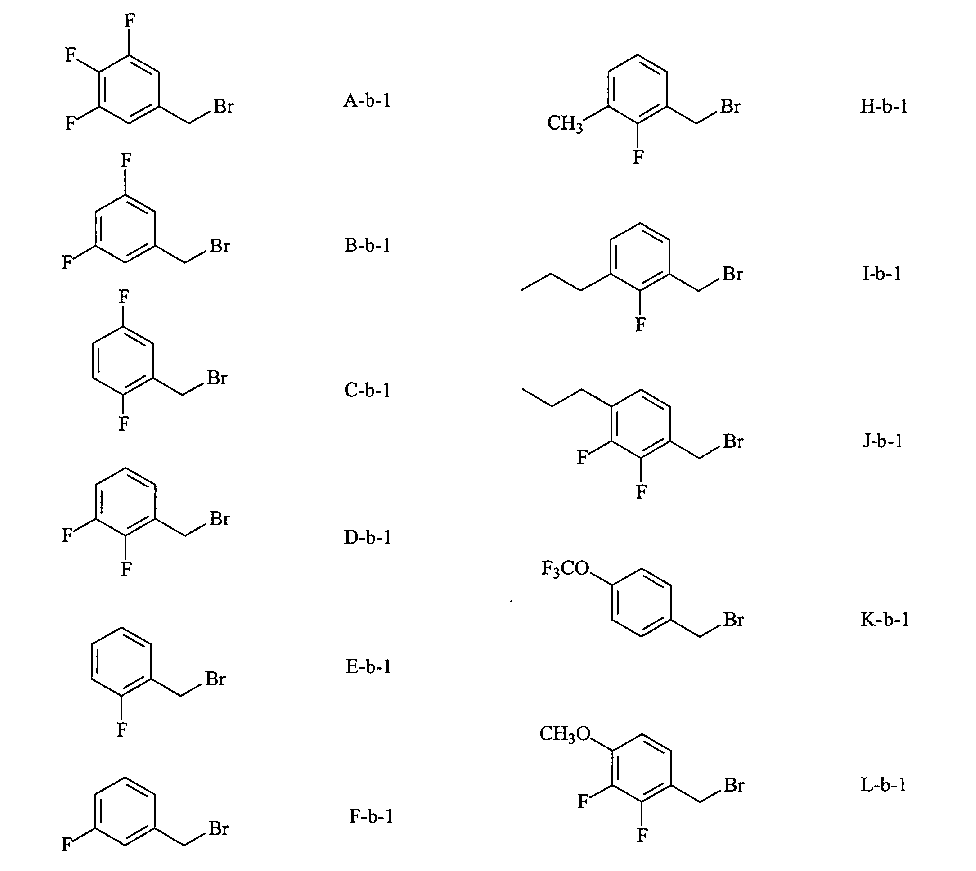

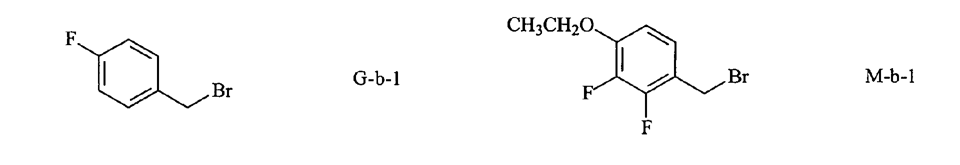

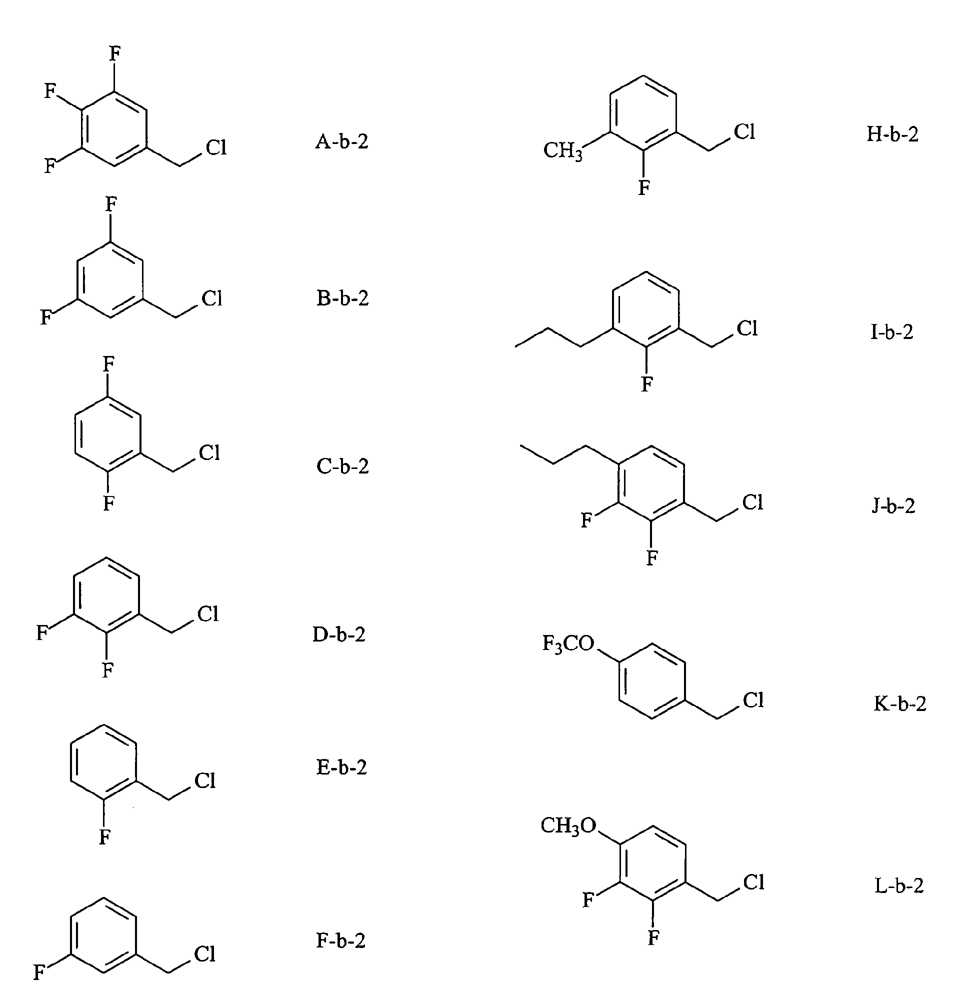

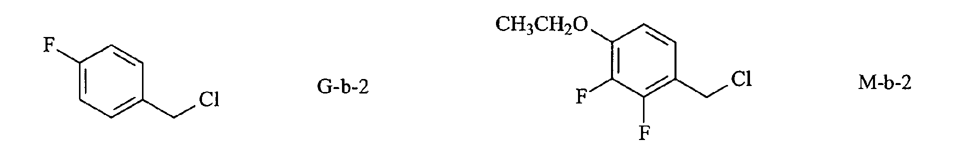

Among these compounds, preferred are benzyl bromide compounds such as the aforementioned A-b-1 to M-b-1 in having superior reactivity.

Furthermore, in order to produce the p-type liquid crystalline compounds, A-b-1 and B-b-1 are preferable, in particular the A-b-1 is preferable due to its superior reactivity.

On the other hand, in order to produce the n-type liquid crystalline compounds, the D-b-1,J-b-1,L-b-1,M-b-1 are preferable.

Suitable examples of the cyclohexyl carbonyl chloride compound, as another starting material of the present invention, are represented by the following formula (5), in which X1, R2, and 1 are defined as in formula (1). 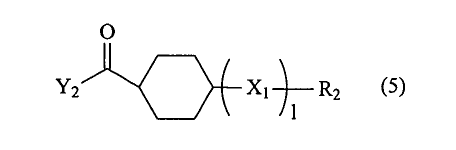 specific examples of the cyclohexyl carbonyl chloride compound include those represented by formulae ch-1 to ch-15 shown below, in which R2 is defined as in formula (1) and Y2 denotes halogen atoms such as chlorine atomes and bromine atom.

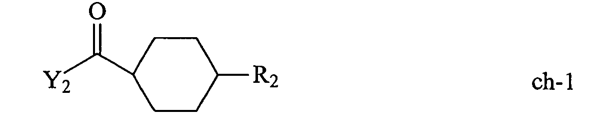

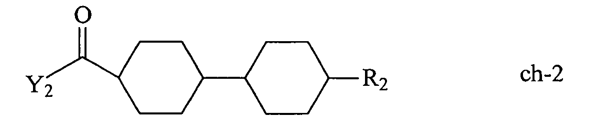

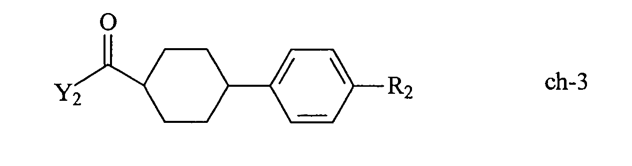

Furthermore, among these ch-1 to ch-3, ch-1 and ch-2 are particularly preferred in scope of anisotropy in a tetralin compound thereof.

Specific examples of particular preferable compounds of ch-1 and ch-2 include those represented by formula ch-1-a to ch-2-h shown below.

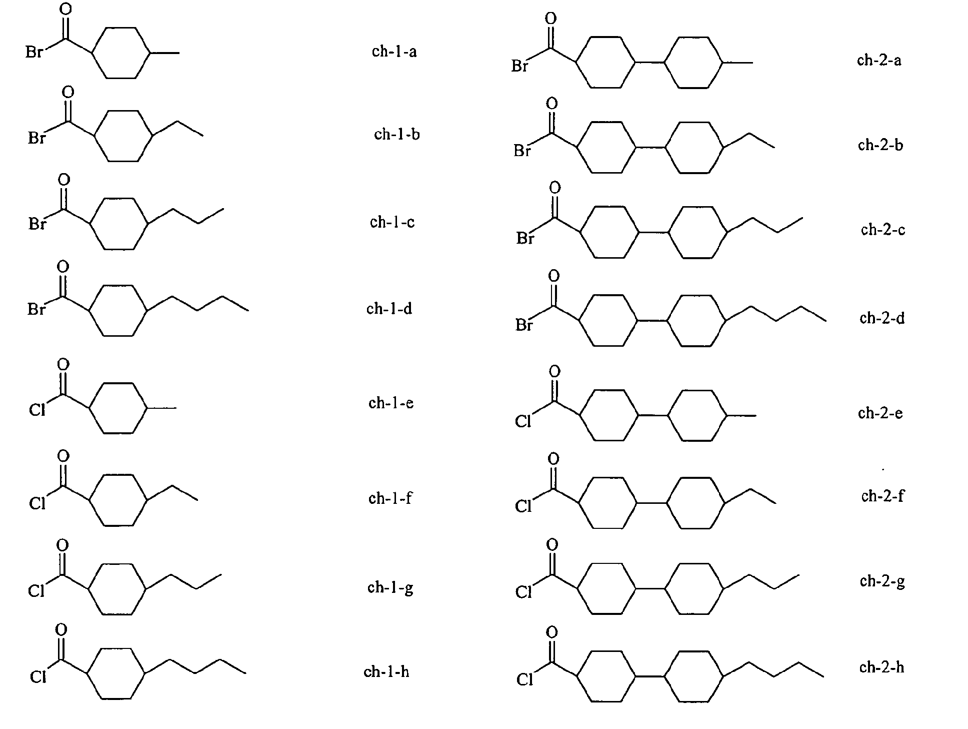

As mentioned above, the process of the present invention is carried out in the presence of a Pd catalyst and zinc powder. Here, the Pd catalyst, conventional Pd catalysts such as, palladium halides, sulphates, phosphates can be arbitrarily used. However, in the present invention, palladium-(II) catalyst having triphenylphosphine as ligands is preferred in that the catalyst exhibits excellent activity in spite that the triphenylphosphine is a quite low-cost material.

Examples of palladium-(II) catalyst having triphenylphosphine as a ligand include tetrakis(triphenylphosphine)palladium(0), bis(triphenylphosphine)palladium-(II)-chloride, and palladium-(II)-acetate having triphenylphosphine. Among these the catalysts, in particular, palladium-(II)-acetate having triphenylphosphine is preferred.

Further, the palladium-(II) catalyst is used as a complex with zinc powder. The catalyst is highly activated thereby.

The preferable diameter of the zinc powder is within the range of 5 to 10 µm, so that the high yield and selectivity can be achieved.

The reaction is advantageously carried out in an oxgen-free atmosphere, whereby the production of by-products can be effectively avoided. In concrete terms, the reaction can be carried out with suppling inert gas such as nitrogen and carbon dioxide into reaction vessel so as to keep less than 1% by weight of oxygen content.

Further, it is preferable that the reaction is carried out by controlling the reaction temperature within the range of -5°C to 25°C. The process of the present invention is especially characterized in that the reaction shall be carried out at a temperature within the range, so that high yield and selectivity of the objective benzyl ketone compound can be achieved in spite of using the fluorine atom contained benzyl haloid which expected for the skilled person as a quite weak reaction reagent due to the strong electron attracting property of the fluorine atom.

The solvents can be arbitrarily used in the reaction, if necessary. Examples of preferable solvents include ethyleneglycol dimethylether and ethyleneglycol diethylether.

The reaction can be advantageously carried out by the following method. For example, in a reaction vessel were placed a predetermined amount of catalyst, triphenylphosphine as needed, and zinc powder. Then, the contents were evacuated with a pump, and were purged with inert gas such as mentioned above. Preferably, the procedure of evacuation and purging may be repeated several times until the concentration of oxygen is below 1% by volume.

Next, a dried organic solvent may be added to the reaction vessel, preferably, then the reaction vessel is cooled so that the temperature of the content is within the range of -5°C to 10°C.

A mixture of the fluorine atom containing benzyl haloid and the cyclohexyl carbonyl chloride compound may be added into the reaction vessel dropwisely or continually. Here, it is preferable that additon speed of the mixture is controlled so that the reaction temperature keeps a constant level.

Afterwards, the mixture is stirred at the same temperature until when the reaction completed, thereby the objective product can be obtained.

The products obtained according to the method of the invention may be purified according to the customary techniques used by persons skilled in the art. For example, after warming the product to room temperature when it was cooled, subjecting the product to filtration in order to remove solid constituents, then washing the liquid phase with a proper solvent. Furthermore, the filtration and washing may be repeated as needed, then by distillation or recrystallisation the objective purified product is obtained.

This invention will be further explained by the following non-limiting examples which are intended to be purely exemplary of this invention.

### EXAMPLES

### EXAMPLE 1

### Synthesis of the following compound.

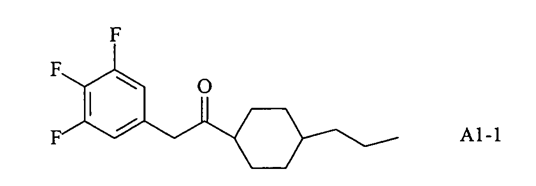

0.44 mmol of Pd-(II)-acetate, 0.88 mmol of triphenylphosphine and 88 mmol of zinc were placed in a reaction vessel. The contents were evacuated with a membrane pump, and were purged with N₂. The procedure was repeated two times in order to remove oxygen completely.

30 ml of ethyleneglycol dimethylether, which was dried by refluxing in the presence of sodium and benzophenone until color changed to blue, and then distilled, was added.

Then, the reaction vessel was cooled to 0°C.

A mixture of 44 mmol of 3,4,5-trifluorobenzyl bromide, and 44 mmol of 4-propylcyclohexylcarbonyl chloride in 30 ml ethyleneglycol dimethylether was slowly added, so that the temperature of the mixture was 0°C.

The mixture was stirred at the same temperature for one hour, then warmed to room temperature, filtrated, the catalyst mixture was washed with diethylether, the solvents were distilled off, diethylether was added, solid parts were separated by filtration, the organic phase was washed with water twice and once with aqueous NaCl-solution.

The solvent was distilled off, the solid was dissolved in methanol, filtrated, and the solvent was distilled off.

The solid was distilled. At 80°C bath temperature, 0.011 mbar, by-product was removed to obtain the objective benzyl ketone compound of the present invention.

Yield and selectivity of the objective benzyl ketone are shown in table1.
**MS** [m/z] 297 [M-1], 153 (Pr-Cyc-CO⁺), 145 (F₃-Aryl-CH₂⁺), 125 (Pr-Cyc⁺), 83 (C₆H₁₁⁺), 69 (C₅H₉⁺), 55 (C₄H₇⁺), 41 (C₃H₅⁺)
^{**1**}**H-NMR** (300 MHz, CDCl₃, δ. [ppm]): 0.80-1.05 (m, 5 H, CH₃/CH₂); 1.10-1.40 (m, 3H, CH₂/CH); 1.42-1.48 (m, 4H, CH₂); 1.76-1.99 (m, 4H CH₂); 2.39 (m, 1H, CH); 3.67 (d, 2H, CH₂); 6.81 (m, 2H, CHₐᵣ)
^{**13**}**C-NMR** (75.4 MHz, CDCl₃, δ. [ppm]): 14.6 (CH₃); 20.1 (CH₂); 28.7 (2xCH₂); 32.5 (2xCH₂); 36.9 (CH₂); 39.7 (CH₂); 46.6 (CH₂); 42.6 (CH); 51.3 (CH); 113.9 (2xCHₐᵣ); 130.8 (Cₐᵣ); 139.3 (CFₐᵣ; 151.3 (2xCFₐᵣ) 209.8 (C=O)
^{**19**}**F-NMR** (282.2 MHz, CDCl₃, δ. [ppm]): -145.9 (2F, m); -174.3 (m)

### EXAMPLE 2

### Synthesis of the following compound

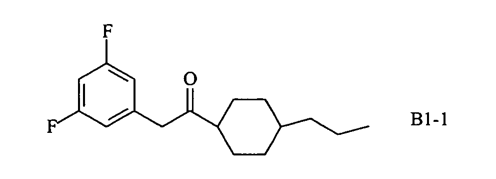

A benzyl compound of the present invention was prepared in the same manner as in Example 1 except for using 3,5-difluoro benzyl bromid as a starting material.
Yield and selectivity of the objective benzyl ketone are shown in table 1.
**MS** [m/z] 153 (Pr-Cyc-CO⁺), 127 (F₂-Aryl-CH₂⁺), 125 (Pr-Cyc⁺), 83 (C₆H₁₁⁺), 69 (C₅H₉⁺), 55 (C₄H₇⁺), 41 (C₃H₅⁺)

### EXAMPLE 3

### Synthesis of the compound B1-1.

A benzyl compound of the present invention was prepared in the same manner as in Example 1 except for using 3,5-difluoro benzyl bromid as a starting material, and controlling temperature in the reaction vassel so as to be at 25 °C. Yield and selectivity of the objective benzyl ketone were shown in table 1.
**MS** [m/z] 153 (Pr-Cyc-CO⁺), 127 (F₂-Aryl-CH₂⁺), 125 (Pr-Cyc⁺), 83 (C₆H₁₁⁺), 69 (C₅H₉⁺), 55 (C₄H₇⁺), 41 (C₃H₅⁺)

### EXAMPLE 4

### Synthesis of the following compound

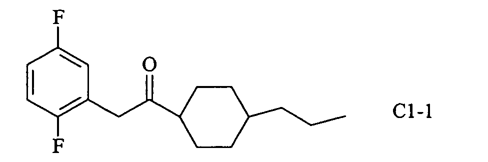

A benzyl compound of the present invention was prepared in the same manner as in Example 1 except for using 2,5-difluoro benzyl bromid as a starting material.
Yield and selectivity of the objective benzyl ketone are shown in table 1.
**MS** [m/z] 153 (Pr-Cyc-CO⁺), 127 (F₂-Aryl-CH₂⁺), 125 (Pr-Cyc⁺), 83 (C₆H₁₁⁺), 69 (C₅H₉⁺), 55 (C₄H₇⁺), 41 (C₃H₅⁺)

### EXAMPLE 5

### Synthesis of the compound C1-1.

A benzyl compound of the present invention was prepared in the same manner as in Example 1 except for using 2,5-difluoro benzyl bromid as a starting material, and controlling temperature in the reaction vessel so as to be at 25 °C.
Yield and selectivity of the objective benzyl ketone are shown in table 1.
**MS** [m/z] 153 (Pr-Cyc-CO⁺) , 127 (F₂-Aryl-CH₂⁺), 125 (Pr-Cyc⁺), 83 (C₆H₁₁⁺), 69 (C₅H₉⁺), 55 (C₄H₇⁺), 41 (C₃H₅⁺)

### EXAMPLE 6

### Synthesis of the compound C1-1.

A benzyl compound of the present invention was prepared in the same manner as in Example 1 except for using 2,5-difluoro benzyl bromid as a starting material, and using 0.44 mmol of bis(triphenylphosphine)palladium-(II)chloride, and 88 mmol of zinc as a catalyst.
Yield and selectivity of the objective benzyl ketone were shown in table 1.
**MS** [m/z] 153 (Pr-Cyc-CO⁺), 127 (F₂-Aryl-CH₂⁺), 125 (Pr-Cyc⁺), 83 (C₆H₁₁⁺), 69 (C₅H₉⁺), 55 (C₉H₇⁺), 41 (C₃H₅⁺)

### EXAMPLE 7

### Synthesis of the following compound

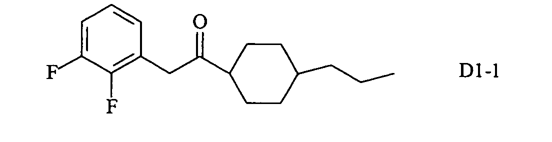

A benzyl compound of the present invention was prepared in the same manner as in Example 1 except for using 2,3-difluoro benzyl bromid as a starting material.
Yield and selectivity of the objective benzyl ketone are shown in table 1.
**MS** [m/z] 153 (Pr-Cyc-CO⁺), 127 (F₂-Aryl-CH₂⁺), 125 (Pr-Cyc⁺), 83 (C₆H₁₁⁺), 69 (C₅H₉⁺), 55 (C₄H₇⁺), 41 (C₃H₅⁺)

### EXAMPLE 8

### Synthesis of the following compound

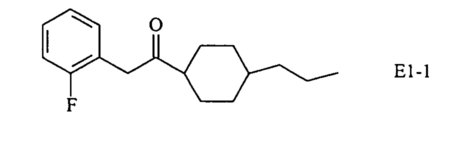

0.02 mmol of Pd-(II)-acetate, 0.46 mmol of triphenylphosphine and 4.5 mmol of zinc were placed in a reaction vessel.
The contents were evacuated with a membrane pump, and were purged with N₂. The procedure was repeated two times in order to remove oxygen completely.

2 ml ethyleneglycol dimethylether, which was dried by refluxing in the presence of sodium and benzophenone until color changed to blue, and then distilled,) was added.

Then, the reaction vessel was cooled to 0°C.

A mixture consisting of 2.12 mmol of 2-fluorobenzyl bromide and 2.13 mmol of 4-propylcyclohexylcarbonyl chloride in 3 ml ethyleneglycol dimethylether was slowly added, so that the temperature of the mixture was 0°C.

The mixture was stirred at the same temperature for one hour, then warmed to room temperature. Afterwards, the objective benzyl ketone was obtained by the same manner as in Example 1. Yield and selectivity of the objective benzyl ketone are shown in table 1.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 9

### Synthesis of the compound E1-1.

A benzyl compound of the present invention was prepared in the same manner as in Example 8 except for controlling temperature in the reaction vessel so as to be at 25 °C.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 10

### Synthesis of the compound E1-1.

A benzyl compound of the present invention was prepared in the same manner as in Example 8 except for using 0.44 mmol of bis(triphenylphosphine)palladium-(II)chloride, and 88 mmol of zinc as a catalyst.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 11

### Synthesis of the compound E1-1.

A benzyl compound of the present invention was prepared in the same manner as in Example 8 except for using 0.44 mmol of tetrakis(triphenylphosphine)palladium(0) and 88 mmol of zinc as a catalyst.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 12

### Synthesis of the following compound F1-1

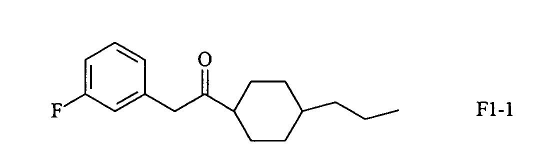

A benzyl compound of the present invention was prepared in the same manner as in Example 1 except for using 2-fluoro-3-methyl benzyl bromide as a starting material.
Yield and selectivity of the objective benzyl ketone are shown in table 1.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 13

### Synthesis of the compound E1-1.

A benzyl compound of the present invention was prepared in the same manner as in Example 12 except for controlling temperature in the reaction vessel so as to be at 25 °C .
Yield and selectivity of the objective benzyl ketone are shown in table1.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 14

### Synthesis of the compound E1-1.

A benzyl compound of the present invention was prepared in the same manner as in Example 12 except for using 0.44 mmol of tetrakis(triphenylphosphine)palladium(0) and 88 mmol of zinc as a catalyst.
Yield and selectivity of the objective benzyl ketone are shown in table 1.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 15

### Synthesis of the following compound

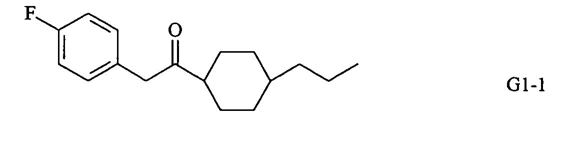

A benzyl compound of the present invention was prepared in the same manner as in Example 1 except for using 4-fluorobenzyl bromid as a starting material.
Yield and selectivity of the objective benzyl ketone are shown in table 1.
**MS** [m/z] 153 (Pr-Cyc-CO⁺), 125 (Pr-Cyc⁺), 109 (F-Aryl-CH₂⁺), 83 (C₆H₁₁⁺), 69 (C₅H₉⁺), 55 (C₄H₇⁺), 41 (C₃H₅⁺)

### EXAMPLE 16

### Synthesis of the following compound

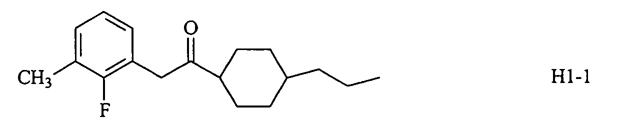

A benzyl compound of the present invention was prepared in the same manner as in Example 1 except for using 1-fluoro-2-methyl benzyl bromid as a starting material.
Yield and selectivity of the objective benzyl ketone are shown in table 1.
**MS** [m/z] 276 [M⁺], 153 (Pr-Cyc-CO⁺), 125 (Pr-Cyc⁺), 123 (F(CH₃)-Aryl-CH₂⁺), 83 (C₆H₁₁⁺), 69 (C₅H₉⁺), 55 (C₄H₇⁺), 41 (C₃H₅⁺)

### EXAMPLE 17

### Synthesis of the compound H1-1.

A benzyl compound of the present invention was prepared in the same manner as in Example 16 except for controlling the temperature in the reaction vessel so as to be at 25 °C.
Yield and selectivity of the objective benzyl ketone are shown in table 1.
**MS** [m/z] 276 [M⁺], 153 (Pr-Cyc-CO⁺), 125 (Pr-Cyc⁺), 123 (F(CH₃)-Aryl-CH₂⁺), 83 (C₆H₁₁⁺), 69 (C₅H₉⁺), 55 (C₄H₇⁺), 41 (C₃H₅⁺)

### EXAMPLE 18

### Synthesis of the following compound

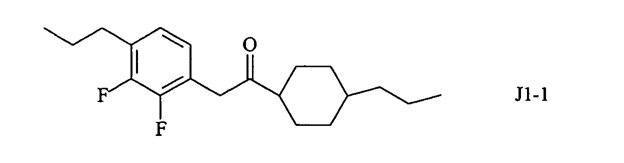

A benzyl compound of the present invention was prepared in the same manner as in Example 1 except for using 2,3-difluoro-4-propylbenzylbromid as a starting material and using 2.2 mmol of bis(triphenylphosphine)palladium-(II)chloride and 88 mmol of zinc as a catalyst.
Yield and selectivity of the objective benzyl ketone are shown in table 1.
**MS** [m/z] 322 [M⁺], 169 (F(C₃H₇)-Aryl-CH₂⁺) 153 (Pr-Cyc-CO⁺), 125 (Pr-Cyc⁺), 83 (C₆H₁₁⁺), 69 (C₅H₉⁺), 55 (C₄H₇⁺), 41 (C₃H₅⁺)

### EXAMPLE 19

### Synthesis of the compound J1-1.

A benzyl compound of the present invention was prepared in the same manner as in Example 18 except for using 0.44 mmol of tetrakis(triphenylphosphine)palladium(0) and 88 mmol of zinc as a catalyst.
Yield and selectivity of the objective benzyl ketone are shown in table 1.
**MS** [m/z] 322 [M⁺], 169 (F(C₃H₇)-Aryl-CH₂⁺) 153 (Pr-Cyc-CO⁺), 125 (Pr-Cyc⁺), 83 (C₆H₁₁⁺), 69 (C₅H₉⁺), 55 (C₄H₇⁺), 41 (C₃H₅⁺)

### EXAMPLE 20

### Synthesis of the following compound

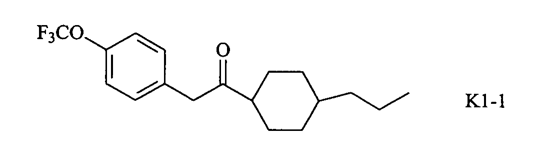

A benzyl compound of the present invention was prepared in the same manner as in Example 1 except for using 4-(trifluoro)methoxy-benzyl bromid as a starting material.
Yield and selectivity of the objective benzyl ketone are shown in table 1.
**MS** [m/z] 175 (F₃CO-Aryl-CH₂⁺) 153 (Pr-Cyc-CO⁺), 125 (Pr-Cyc⁺), 83 (C₆H₁₁⁺), 69 (C₅H₉⁺), 55 (C₄H₇⁺), 41 (C₃H₅⁺)

### EXAMPLE 21

### Synthesis of the compound K1-1.

A benzyl compound of the present invention was prepared in the same manner as in Example 20 except for controlling the temperature in the reaction vessel so as to be at 25 °C.
Yield and selectivity of the objective benzyl ketone are shown in table 1.
**MS** [m/z] 175 (F₃CO-Aryl-CH₂⁺) 153 (Pr-Cyc-CO⁺), 125 (Pr-Cyc⁺), 83 (C₆H₁₁⁺), 69 (C₅H₉⁺), 55 (C₄H₇⁺), 41 (C₃H₅⁺)

### EXAMPLE 22

### Synthesis of the following compound

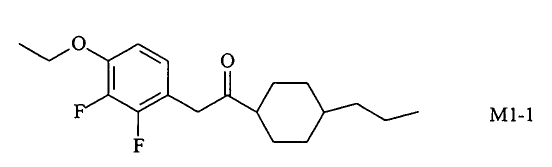

A benzyl compound of the present invention was prepared in the same manner as in Example 1 except for using 2,3-difluoro-4-ethoxy-benzyl bromid as a starting material, using 0.44 mmol of bis(triphenylphosphine)palladium-(II)chloride and 88 mmol of zinc as a catalyst, and controlling the temperature in the reaction vessel so as to be at 25 °C.
Yield and selectivity of the objective benzyl ketone are shown in table 1.
**MS** [m/z] 324 [M⁺], 153 (Pr-Cyc-CO⁺), 143 (OH⁺=(F,F,O=)C₆H₂), 125 (Pr-Cyc⁺), 83 (C₆H₁₁⁺), 69 (C₅H₉⁺), 55 (C₄H₇⁺), 41 (C₃H₅⁺)

### EXAMPLE 23

### Synthesis of the compound M1-1.

A benzyl compound of the present invention was prepared in the same manner as in Example 22 except for using 2.2 mmol of tetrakis(triphenylphosphine)palladium(0) and 88 mmol of zinc as a catalyst.
Yield and selectivity of the objective benzyl ketone are shown in table 1.
**MS** [m/z] 324 [M⁺], 153 (Pr-Cyc-CO⁺), 143 (OH⁺=(F,F,O=)C₆H₂), 125 (Pr-Cyc⁺), 83 (C₆H₁₁⁺), 69 (C₅H₉⁺), 55 (C₄H₇⁺), 41 (C₃H₅⁺)

### EXAMPLE 24

### Synthesis of the following compound

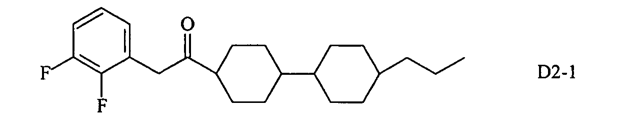

1.0 mmol of Pd-(II)-acetate, 2.0 mmol of triphenylphosphine and 88 mmol of zinc were placed in a reaction vessel. The contents were evacuated with a membrane pump, and were purged with N₂. The procedure was repeated two times in order to remove oxygen completely.

80 ml of ethyleneglycol dimethylether, which was dried by refluxing in the presence of sodium and benzophenone until color changed to blue, and then distilled, was added.

Then, the reaction vessel was cooled to 0°C.

A mixture of 65 mmol of 2,3-difluorobenzyl bromide, and 55 mmol of 4'-propylcyclohexyl-4-cyclohexylcarbonyl chloride in 60 ml ethyleneglycol dimethyl-ether was slowly added, so that the temperature of the mixture was 0°C.

The mixture was stirred at the same temperature for 5 hours, then warmed to room temperature.

Then, the mixture was quenched with diluted HCl and water and extracted into diethylether three times, then the layers were filtrated to remove excess zinc powder before separation of the layers. The organic layers were collected, dried (Na₂SO₄), some of the solvent removed and the product crystallized by cooling to -20°C.

The product was filtered off and washed with a little of cold diethylether and dried. The washings are collected, some of the solvent removed and again crystallized to obtain the objective benzyl ketone compound of the present invention.

Yield and selectivity of the objective benzyl ketone were shown in table1.
**MS** [m/z] (TMS deriv./TMS-enolether) 434 (M⁺+TMS), 307 (C₂₃H₃₂F₂⁺), 255 (C₂₀H₃₅O₂⁺), 73 (TMS⁺, base peak)
^{**1**}**H-NMR** (300 MHz, CDCl₃, δ.[ppm]): 0.80-1.20 (m, 20H, CH/CH₂); 0.87 (t, 3H, ³J_{HH}=7.1 Hz, CH₃); 1.20-1.44 (m, 5H, CH₂/CH); 1.64-1.90 (m, 8H, CH/CH₂); 1.92-2.00 (m, 2H CH₂); 2.37-2.46 (m, 1H, CH); 2.79 (d, 2H, ⁴J_{HF}=1.46 Hz, CH₂); 6.86-6.94 (m, 1H, CHₐᵣ); 6.98-7.11 (m, 2H, CHₐᵣ)
^{**13**}**C-NMR** (75.4 MHz, CDCl₃, δ.[ppm]): 14.4 (CH₃); 20.3 (CH₂); 28.8 (2xCH₂); 29.3 (2xCH₂); 30.0 (2xCH₂); 33.6 (2xCH₂); 37.5 (CH₂); 39.8 (CH); 40.5 (CH₂, t, J=2.23 Hz); 42.6 (CH); 43.2 (CH); 51.0 (CH); 116.0 (CHₐᵣ, d, J=17.1); 123.9 (CHₐᵣ, dd); 124.3 (Cₐᵣ, d); 126.4 (CHₐᵣ, dd); 148.1 (CFₐᵣ, dd, ¹J_{CF}=120.4 Hz, ²J_{CF}=13.2 Hz); 151.3 (CFₐᵣ, dd, ¹J_{CF}=121.1 Hz, ²J_{CF}=13.1 Hz); 209.3 (C=O)
^{**19**}**F-NMR** (282.2 MHz, CDCl₃, δ.[ppm]): -149.6 (m); -153.5 (m)

### COMPARATIVE EXAMPLE 1

### Synthesis of the following compound

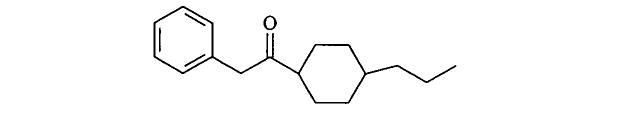

A benzyl compound of was prepared in the same manner as in Example 1 except for using benzyl bromid as a starting material, and controlling the temperature in the reaction vessel so as to be at 25 °C .
Yield and selectivity of the benzyl ketone arere shown in table 1.

### COMPARATIVE EXAMPLE 2

### Synthesis of the following structure compound in the presence of metallic nickel.

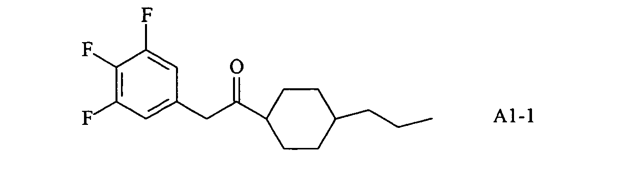

A mixture of 0.2 g (28.5 mmol) lithium wire, 4.2 g (13.4 mmol) NiI₂, and 0.3 g (2.3 mmol) naphthalene in 25 ml ethyleneglycol dimethylether, which was dried by refluxing in the presence of sodium and benzophenone until color changed to blue, and then distilled, under a stream of argon were heated at 50°C in an ultrasonic bath until the lithium wire had disappeared and black metallic nickel was formed.

Then, the mixture was heated to 85°C and a solution of 1.8 g (10.0 mmol) of 4,5-trifluorobenzyl bromide, and 2.0 g (10.6 mmol) of of 4-propylcyclohexylcarbonyl chloride in 10 ml ethyleneglycol dimethylether were added dropwise within 30 min. The mixture was stirred for 30 min at the same temperature. A small sample was taken, one drop water was added, and the mixture was analyzed by GC/MS.
Yield of the target compound was 69%, and as a sole by-product, the compound represented by the following structure was obtained in 9% yield.

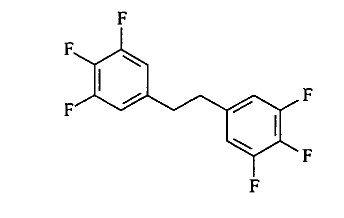

### COMPARATIVE EXAMPLE 3

### Synthesis of the Grignard compound of 3,4,5-trifluorobenzylchloride using

0.8 g (1.9 mmol) Mg-anthracene-THF complex represented by the following formula 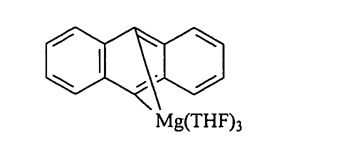 in 5 ml THF (dried over molecular sieve) was cooled to 0°C, and nitrogen was bubbled through the mixture.

Then, 0.4 g (2.2 mmol) of trifluorobenzylchloride was slowly added dropwise. After completion, the mixture was stirred at room temperature for two hours. A small sample was taken, one drop water was added, and the mixture was analyzed by GC/MS.

The 82% of the obtained sample remained as trifluorobenzylchloride, the rest was bis(trifluorophenyl methane).
Therefore, the production of grignard reagents was only observed in a small amount.

**Table 1**

| EXAMPLE No. | cat. | mol-% [cat] | T [°C] | **Yield** [%] | **Selectivity** [%]⁴⁾ |
|---|---|---|---|---|---|
| EXAMPLE 1 | PdOAc/TPP³⁾ | 1 | 0 | 84 (73⁵⁾ | 84 |
| EXAMPLE 2 | PdOAc/TPP³ | 1 | 0 | 69 | 73 |
| EXAMPLE 3 | PdOAc/TPP³⁾ | 1 | r.t. | 76 | 76 |
| EXAMPLE 4 | PdOAc/TPP³⁾ | 1 | 0 | 77 | 77 |
| EXAMPLE 5 | PdOAc/TPP³⁾ | 1 | r.t. | 63 | 63 |
| EXAMPLE 6 | BPdCl²⁾ | 1 | 0 | 74 | 74 |
| EXAMPLE 7 | PdOAc/TPP³ | 1 | 0 | 67 | 71 |
| EXAMPLE 8 | PdOAc/TPP³⁾ | 1 | 0 | 85 | 85 |
| EXAMPLE 9 | PdOAc/TPP³⁾ | 1 | r.t. | 55 | 57 |
| EXAMPLE 10 | BPdCl²⁾ | 1 | 0 | 85 | 85 |
| EXAMPLE 11 | TkPd¹⁾ | 1 | 0 | 80 | 80 |
| EXAMPLE 12 | PdOAc/TPP³⁾ | 1 | 0 | 87 | 87 |
| EXAMPLE 13 | PdOAc/TPP³⁾ | 1 | r.t. | 78 | 78 |
| EXAMPLE 14 | TkPd¹⁾ | 1 | 0 | 88 | 88 |
| EXAMPLE 15 | PdOAc/TPP³⁾ | 1 | 0 | 79 | 79 |
| EXAMPLE 16 | PdOAc/TPP³ | 1 | 0 | 62 | 65 |
| EXAMPLE 17 | PdOAc/TPP³⁾ | 1 | r.t. | 25 | 26 |
| EXAMPLE 18 | PdOAc/TPP³⁾ | 1 | r.t. | 41 | 41 |
| EXAMPLE 19 | TkPd¹⁾ | 1 | r.t. | 33 | 33 |
| EXAMPLE 20 | PdOAc/TPP³⁾ | 1 | 0 | 86 | 86 |
| EXAMPLE 21 | PdOAc/TPP³⁾ | 1 | r.t. | 73 | 73 |
| EXAMPLE 22 | BPdCl²⁾ | 1 | r.t. | 33 | 33 |
| EXAMPLE 23 | TkPd¹⁾ | 5 | r.t. | 48 | 48 |
| EXAMPLE24 | PdOAc/TPP³⁾ | 2 | 0 | 87 (73⁵⁾ | 95 |
| COMPARATIVE EX.1 | PdOAc/TPP³⁾ | 1 | r.t. | 63 | 63 |

| | | | | | |
|---|---|---|---|---|---|
| ¹⁾TkPd: tetrakis(triphenylphosphine)palladium(0) | | | | | |
| ²⁾BPCl: bis(triphenylphosphine)palladium-(II)-chloride | | | | | |
| ³⁾PdOAc: palladium-(II)-acetate, TPP: Triphenylphosphine, PdOAc/TPP = 1/2 | | | | | |
| ⁴⁾ investigated by GC/MS; missing parts to 100 % are further by-products in low amounts. | | | | | |
| ⁵⁾ isolated yield | | | | | |

## Claims

1. A fluorine atom containing benzyl ketone compound represented by the following formula (1), 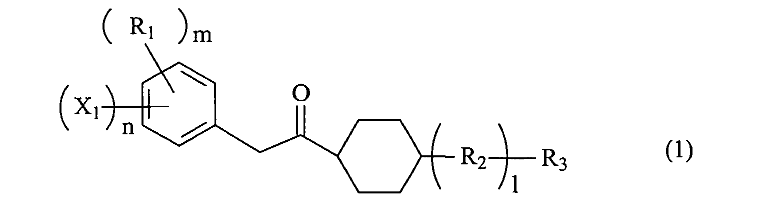 wherein R1 denotes methyl, ethyl, propyl, methoxy, or ethoxy, X1 denotes a fluorine atom or tri-fluoro methoxy, R2 denotes 1,4-cyclohexylene, or 1,4-phenylene, R3 denotes an aliphatic hydrocarbon group having 1 to 10 carbon atoms, l means an integer of 0 or l, m means an integer of 0 or 1, and n means an integer of 1 to 3.

2. A fluorine atom containing benzyl ketone compound according to claim 1, wherein the compound is represented by the following formula (2), 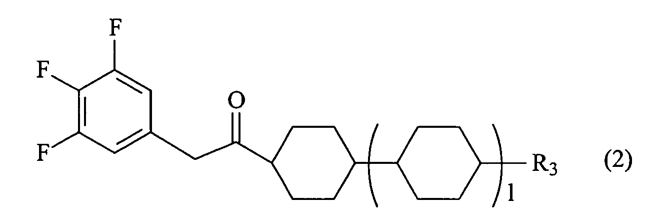 wherein R3 denotes an linear or branched aliphatic hydrocarbon group having 1 to 10 carbon atoms, and 1 means an integer of 0 or 1.

3. A fluorine atom containing benzyl ketone compound according to claim 1, wherein the compound is represented by the following formula (3), 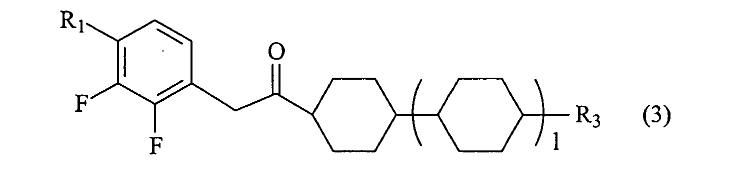 wherein R1 denotes methyl, ethyl, propyl, methoxy, or ethoxy,R3 denotes an aliphatic hydrocarbon group having 1 to 10 carbon atoms, and l means an integer of 0 or 1.

4. A process for producing a fluorine atom containing benzyl ketone compound comprising; reacting a fluorine atom containing benzyl haloid with an aliphatic hydrocarbon group containing cyclohexyl carbonyl chloride in the presence of Pd catalyst and zinc powder under an of oxgen-free atmosphere.

5. A process for producing a fluorine atom containing benzyl ketone compound according to claim 4, wherein the reaction is carried out at a temperature within the range of -5°C to 25°C.

6. A process for producing a fluorine atom containing benzyl ketone compound according to claim 4, wherein the fluorine atom containing aryl methyl haloid is tri-fluorobenzl bromide.

7. A process for producing a fluorine atom containing benzyl ketone compound according to claim 4, wherein the Pd catalyst comprises a Pd (*II* ) compound and triphenyl phoshines.

8. A process for producing a fluorine atom containing benzyl ketone compound according to claim 7, wherein the Pd catalyst comprises a Pd (*II* ) acetate and triphenyl phoshines.

9. A process for producing a fluorine atom containing benzyl ketone compound according to claim 4 to 8, wherein the amount of the Pd catalyst is within the range of 0.5 to 5 % by mol based on the total amount of starting materials.
